# EUROPEAN PATENT SPECIFICATION

(11) **EP 1 802 581 B1**
(45) Date of publication and mention of the grant of the patent: **02.04.2008**
(21) Application number: 05820982.6
(22) Date of filing: 21.10.2005
(51) Int. Cl.: C07D 215/44, A61K 31/4706, A61P 9/10

(54) **4 [(2,4-DICHLORO-5-METHOXYPHENYL)AMINO]-6-ALKOXY-7-ETHYNYL-3-QUINOLINECARBONITRILES FOR THE TREATMENT OF ISCHEMIC INJURY**
4 [(2,4-DICHLORO-5-METHOXYPHENYL)AMINO]-6-ALKOXY-7-ETHYNYL-3-QUINOLINECARBONITRILE ZUR BEHANDLUNG VON ISCHÄMIE
4 [(2,4-DICHLORO-5-METHOXYPHENYL)AMINO]-6-ALKOXY-7-ETHYNYL-3-QUINOLINECARBONITRILES POUR TRAITER UNE LESION ISCHEMIQUE

(30) Priority: 22.10.2004 US 621280 P
(43) Date of publication of application: 04.07.2007
(73) Proprietor: Wyeth, Madison, NJ 07940 (US)
(72) Inventor: BOSCHELLI, Diane, Harris, New City, New York 10956 (US); ZALESKA, Margaret, Narberth, PA 19072 (US); BARRIOS-SOSA, Ana Carolina, Olanta, SC 29114 (US)
(74) Representative: Mannion, Sally Kim
(86) International application number: PCT/US2005/037839
(87) International publication number: WO 2006/047262

(56) References cited:
- WO-A-20/04075898
- US-B1- 6 689 772
- BARRIOS SOSA, ANA CAROLINA ET AL: "Synthesis and inhibition of Src kinase activity by 7-ethenyl and 7-ethynyl-4-anilino-3-quinolinecarbonitril es" BIOORGANIC & MEDICINAL CHEMISTRY LETTERS, ( CODEN: BMCLE8), vol. 14, no. 9, 2004, pages 2155-2158, XP002368408 ISSN: 0960-894X

## Description

The present invention relates to 4-[(2,4-dichloro-5-methoxyphenyl)amino]-6-alkoxy-7-ethynyl-3-quinolinecarbonitriles and to methods for using them in the treatment of ischemic injury.

### BACKGROUND OF THE INVENTION

Stroke is the third leading cause of death and the major cause of disability in the US, where approximately 750,000 strokes occur each year. Ischemic stroke comprises about 80% of this number, with primary intracerebral hemorrhagic stroke about 15-20%. To date, the only approved efficacious treatment for acute ischemic cerebral infarction is thrombolytic therapy by means of intravenous administration of t-PA, recombinant tissue plasminogen activator. The usefulness of this therapy is extremely limited. It must be given within a three hour window after the onset of symptoms, while a majority of patients seek and/or receive treatment after a substantial delay. In addition, treatment with t-PA carries an increased risk of causing intracerebral hemorrhage, a potentially devastating complication. Presence of hemorrhage must be ruled out prior to treatment and blood pressure must be carefully managed and monitored during and after treatment with t-PA. Currently, no neuroprotective therapy is available for treatment of ischemic stroke, hemorrhagic stroke or brain trauma. New treatments for stroke and other conditions associated with vascular permeability are greatly needed.

### DESCRIPTION OF THE INVENTION

In accordance with one aspect of the present invention there are provided compounds of the structural formula: 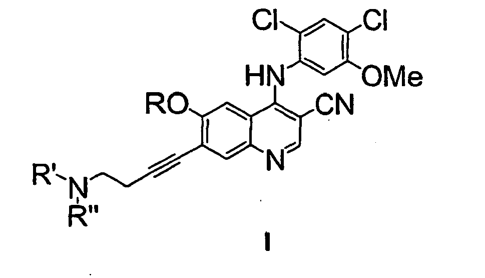 wherein:
R is methyl or ethyl;
R' and R" are independently alkyl of 1 to 3 carbon atoms, or R' and R", taken together with the nitrogen to which they are attached, can form a 5 or 6 membered saturated ring which may optionally contain an additional heteroatom selected from NR"', O or S(O)ₙ;
n is 0-2;
and
R'" is hydrogen or alkyl of 1 to 3 carbon atoms; and pharmaceutically acceptable salts thereof.

In certain preferred embodiments of the invention, R' and R", taken together with the nitrogen to which they are attached, form a N-(C₁-C₃)-alkyl piperazine, piperidine, piperazine or morpholine.

In some preferred embodiments of the invention, R is methyl.

In other preferred embodiments of the present invention R' and R", taken together with the nitrogen atom to which they are attached, form a N-(C₁-C₃)-alkyl piperazine, piperazine or morpholine ring.

Where R' and R" taken together with the nitrogen to which they are attached form a N-(C₁-C₃)-alkyl piperazine, preferably they form N-methylpiperazine.

In still other preferred embodiments of the present invention R' and R" are methyl.

Pharmaceutically acceptable salts are those derived from organic and inorganic acids such as: acetic, lactic, carboxylic, citric, cinnamic, tartaric, succinic, fumaric, maleic, malonic, mandelic, malic, oxalic, propionic, hydrochloric, hydrobromic, phosphoric, nitric, sulfuric, glycolic, pyruvic, methanesulfonic, ethanesulfonic, toluenesulfonic, salicylic, benzoic, and similarly known pharmaceutically acceptable acids.

Five or six membered saturated rings optionally containing one additional heteroatom selected from NR"', O or S(O)ₙ as defined by N R'R" include, but are not limited to, pyrrolidine, pyrazolidine, imidazolidine, piperidine, piperazine, morpholine, thiomorpholine, 1-oxo-1-thiomorpholine and 1,1-dioxo-1-thiomorpholine.

Specific compounds of the invention include:
4-[(2,4-Dichloro-5-methoxyphenyl)amino]-7-[4-(dimethylamino)but-1-ynyl]-6 methoxy-3-quinolinecarbonitrile;
4-[(2,4-Dichloro-5-methoxyphenyl)amino]- 6-methoxy-7-[4-(4-methylpiperazin-1-yl)but-1-ynyl]-3-quinolinecarbonitrile;
4-[(2,4-Dichloro-5-methoxyphenyl)amino]- 6-methoxy-7-(4-morpholin-4-ylbut-1-ynyl)-3-quinolinecarbonitrile;
4-[(2,4-Dichloro-5-methoxyphenyl)amino]- 6-methoxy-7-(4-piperazin-1-ylbut-1-ynyl)-3-quinolinecarbonitrile; and
4-[(2,4-Dichloro-5-methoxyphenyl)amino]- 6-ethoxy-7-[4-(4-methylpiperazin-1-yl)but-1-ynyl]-3-quinolinecarbonitrile, and pharmaceutically acceptable salts thereof.

Other aspects of the invention include pharmaceutical compositions comprising the compounds described herein and at least one pharmaceutically acceptable carrier or excipient, and methods of treating ischemic injury using these compounds.

The compounds of the invention are prepared as illustrated below. The compounds of this invention were prepared from: (a) commercially available starting materials (b) known starting materials which can be prepared as described in literature procedures or (c) new intermediates described in the schemes and experimental procedures herein.

Reactions are performed in a solvent appropriate to the reagents and materials employed and suitable for the transformation being effected. It is understood by those skilled in the art of organic synthesis that the various functionalities present on the molecule must be consistent with the chemical transformations proposed. When not specified, order of synthetic steps, choice of protecting groups and deprotection conditions will be readily apparent to those skilled in the art. In addition, in some instances, substituents on the starting materials may be incompatible with certain reaction conditions. Restrictions pertinent to given substituents will be apparent to one skilled in the art. Reactions were run under inert atmospheres where appropriate.

Compounds of Formula I are prepared as depicted in Scheme 1. Compounds of Formula I wherein R is Me are readily obtained starting from 1a, 3-cyano-4-[(2,4-dichloro-5-methoxyphenyl)amino]-6-methoxy-7-quinolinyl trifluoromethanesulfonate. The preparation of 1a has been reported in the literature, Berger, D. et. al. Bioorg. Med.Chem.Lett. 12, 2761 (2002) and US6521618 (Feb 18, 2003), incorporated by reference herein in their entirety.

Treatment of 1a with 3-butyn-1-ol in the presence of a palladium catalyst, preferably tetrakis(triphenylphosphine)palladium, with copper iodide, in a solvent system such as triethylamine and dioxane at elevated temperature, preferably 95-100°C, provides compounds of formula 2. Reaction of compounds of formula 2 with methanesulfonyl chloride in a solvent system such as N,N-dimethylformamide and tetrahydrofuran at reduced temperature, preferably 0°C, gives the corresponding mesylate. The intermediate mesylate is not normally isolated but is treated directly with an appropriate amine of formula R"R'NH to provide the compounds of formula 1. Other leaving groups such as tosylate can be employed as an alternative to the mesylate group.

In addition to 1a, other starting materials including 1b, 4-[(2,4-dichloro-5-methoxyphenyl)amino]-7-iodo-6-methoxy-3-quinolinecarbonitrile, and 1c, 7-bromo-4-[(2,4-dichloro-5-methoxyphenyl)amino]-6-methoxy-3-quinolinecarbonitrile, can be used to obtain the compounds of formula I where R is Me.

Compounds of Formula I wherein R is Et are readily obtained starting from 4-[(2,4-dichloro-5-methoxyphenyl)amino]-6-ethoxy-7-iodo-3-quinolinecarbonitrile, 1d.

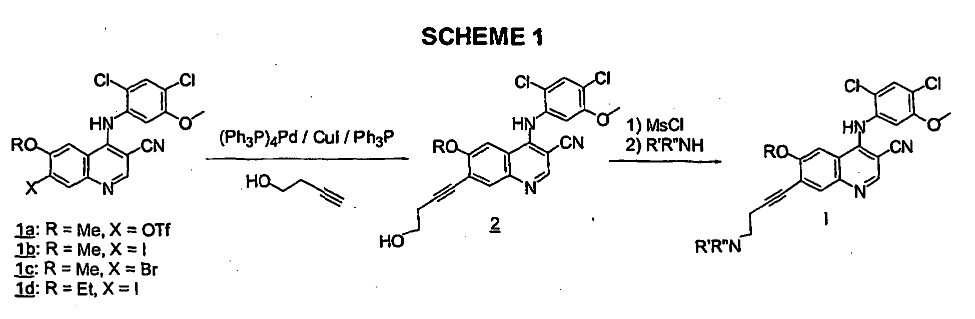

An alternate route to compounds of formula I is depicted in Scheme 2. Compounds of formula 1a-d are treated with an amine of formula 3 in the presence of a palladium catalyst, preferably dichlorobis(triphenylphosphine)palladium(II), with copper iodide and triphenylphosphine, in a solvent system such as triethylamine and N-methylpyrrolidinone at elevated temperature, preferably 70°C, to provide compounds of formula I. Alternatively the reaction of compounds of formula 1a-d with amines of formula 3 can be performed in a solvent system of triethylamine and N,N-dimethylformamide or alternatively in the presence of a base such as potassium carbonate in a solvent system of methanol and tetrahydrofuran.

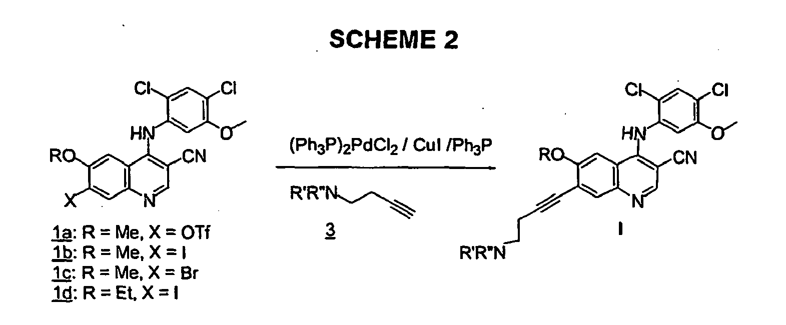

Compounds of the present invention were evaluated in several standard pharmacological tests that showed that compounds of the present invention inhibit Src kinase and are useful for the prevention of vascular permeability.

### Src Kinase Assay

Recombinant human Src enzyme was obtained from PanVera (P3044). Biotinylated peptide corresponding to residues 6 - 20 of Cdk1 was used as a substrate (Biotin-KVEKIGEGTYGVVYK-COOH). Homogeneous fluorescence resonance energy transfer kinase assays were performed using the europium/APC detection format (LANCE, Perkin Elmer). Src enzyme (10 ng) was mixed with biotinylated peptide (final concentration 2 µM), 50 mM Hepes (pH 7.5), 10 mM MgCl₂, 20 µg/ml BSA, 0.001 % Brij-35 (Sigma), 100 µM ATP, 1% DMSO. The kinase reaction was incubated for 70 min at 37°C. The reaction was stopped with EDTA at a final concentration of 30mM EDTA/25mM Hepes (pH 7.5)/10 µg/ml BSA. The mixture was combined with Eu-labeled anti-phosphotyrosine antibody PT66 (Perkin Elmer, AD0068) and Streptavidin Surelight-APC (Perkin Elmer, CR130-100) in 50 mM Hepes (pH 7.5)/20 µg/ml BSA, and incubated for 30 min according to manufacturer's specifications. Fluorescence intensity at 665 nm was used to monitor the extent of the kinase reaction. Multiple entries for a given compound indicate that it was tested multiple times. The results obtained for representative compounds of this invention are listed in Table 1.

### Anchorage Independent Src-transformed Fibroblast Proliferation Assay

Rat2 fibroblasts stably transformed with a plasmid containing a CMV promotor controlled v-Src/Hu c-Src fusion gene in which the catalytic domain of human c-Src was inserted in place of the v-Src catalytic domain in the v-Src gene are used for the measurement of src dependent suspension growth. Ultra-low cluster plates (Costar # 3474) are seeded with 10,000 cells per well on Day 1. Alternatively, Ultra-low cluster plates (Costar 3474) treated with Sigmacote (Sigma), rinsed with 70% ethanol, after drying in the hood, are seeded with 5000 cells. Compound is added in serial two-fold dilutions from 10 micromolar to 0.009 micromolar on Day 2 and MTS reagent (Promega) is added on Day 5 (100 microliters of MTS/medium mix + 100 microliters of medium already on the cells and the absorbance is measured at 490nm. The results are analyzed as follows to yield an IC₅₀ for proliferation (micromolar units) as follows: %inhibition = (Abs490 nm sample - blank)/(Abs490 nm no cmpd control - blank) X 100%. Multiple entries for a given compound indicate that it was tested multiple times.The results obtained for representative compounds of this invention are listed in Table 1.

**Table 1. Inhibition of Src enzymatic and cellular activity**

| | | | | |
|---|---|---|---|---|
| 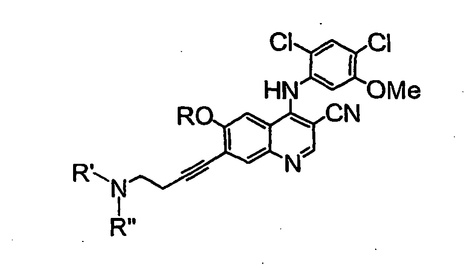 | | | | |

| EXAMPLE | R | NR'R" | Src enzyme IC₅₀ nM | Src cell IC₅₀ nM |
|---|---|---|---|---|
| 1 | Me | NMe₂ | 5.0, 4.7 | 252, 240 |
| 2 | Me | N-Me-piperazine | 3.5, 4.2 | 71, 70, 77 |
| 3 | Me | morpholine | 4.0, 4.1 | 206, 183 |
| 4 | Me | piperazine | 5.9, 4.8 | 435, 605 |
| 5 | Et | N-Me-piperazine | 6.5,2.8 | 96, 183, 154, 103 |

### IV administration of Example 2 provides neuroprotection in a model of transient focal ischemia

Example 2 was tested in a rat model of transient focal ischemia. Wistar rats were subjected to a 90 min occlusion of the middle cerebral artery (MCA) using an intraluminal suture approach as described by Longa et al., Stroke 1989, 20:84 followed by reperfusion for 48 hours. Four hours after the initial onset of ischemia, animals received compound of Example 2 as a single i.v. bolus in 5% dextrose, 0.9% lactic acid at pH 4.5-5.0 (3 mg/kg, 10 mg/kg or 30 mg/kg). Following reperfusion, the animals were evaluated over a 48 hour period for neurological function deficit. Infarct size was measured following sacrifice at 48 hours post MCA occlusion. 10 and 30 mg/kg doses of Example 2 significantly improved recovery from stroke-induced neurological deficits 35% and 53%, respectively as a percent of control (statistical error: 10-12%). Statistically significant reductions in the volume of infarcted brain tissue were observed at 10 mg/kg and 30 mg/kg of Example 2, showing a 32% and 40% reduction in infarct volume as a percent of control (statistical error; 7-10%).

### IV administration of Example 2 provides neuroprotection and improves long-term neurological recovery in a model of permanent focal ischemia

Example 2 was tested in a rat model of permanent occlusion of middle cerebral artery, without the reperfusion, substantially as described by Chen et al., Stroke, 1986,17:738. This is a more stringent model than the transient focal ischemia. Two hours following an electrocoagulation of middle cerebral artery, vehicle or Example 2 were administered at 10 mg/kg i.v followed by 3 additional doses of 10 mg/kg iv administered at 4, 24 and 26 hours post-induction of stroke. In one study, volume of infracted brain tissue was evaluated at 48 hour post-stroke. In a second study, animals were subjected to testing of sensori-motor function based on tactile and propioceptive limb placement substantially as described by DeRyck et al., Brain Res. 1992, 573:44 at three day intervals over a timecourse of three weeks following stroke. In the animals treated with Example 2 a statistically significant reduction by 24% of the infarct volume was found at 48 hours post-induction of stroke. In addition, Example 2 significantly improved long-term sensorimotor recovery from neurological deficits induced in this stroke model.

### IV administration of Example 2 provides neuroprotection in the model of hemorrhagic stroke

Example 2 was tested in a rat model of intracerebral hemorrhage induced by intrastriatal infusion of collagenase substantially as described by Rosenberg et al., Stroke, 1990, 20:801*.* In this model, infusion of collagenase into caudate nucleus leads to proteolytic destruction of collagen in basal lamina of blood vessels and induces intracranial bleeding and edema peaking in 24-48 hours. Example 2 was administered at 4 hours post-induction of hemorrhagic stroke as a single i.v injection at the doses of 10 mg/kg or 30 mg/kg. 24 hours later brain water content was determined as a measure of edema. Results indicate that Example 2 significantly reduces post-hemorrhagic brain edema at both tested doses by 18% and 24% respectively.

Vascular permeability due to disease, injury, or other trauma, may occur in a variety of tissues and organs including organs of the central nervous system, cardiopulmonary system, gastrointestinal system and renal system. Compounds of the present invention are useful for inhibiting vascular permeability caused by disease, injury, or other trauma. In particular, vascular permeability may be inhibited in cerebral and spinal tissue following cerebrovascular events. Vascular permeability is a major cause of vascular leakage and/or edema following a cerebrovascular event and often leads to neurological disorders and disabilities. Cerebrovascular events including, but not limited to transient and acute ischemic events, may be treated in accordance with the present invention. Acute events include, but are not limited to, stroke, head trauma, spinal trauma, general anoxia, hypoxia including fetal hypoxia, hypoglycemia, hypotension as well as similar injuries seen during procedures from embole, hyperfusion and hypoxia. Stroke includes, but is not limited to focal and global ischemia, transient cerebral ischemic attacks, and other cerebral vascular problems accompanied by cerebral ischemia. The instant invention would also be useful for a range of cerebrovascular events including cerebral hemmorhage, infarction due to embolism or thrombosis of the intra- or extra cranial arteries, perinatal asphyxia, in cardiac arrest and status epilepticus, especially where blood flow to the brain is halted for a period of time. Cerebrovascular events associated with vascular leakage also include infections, including, but not limited to encephalitis and meningitis associated with neuroinflammation, which, through vascular leakage propagate injury to surrounding tissues. Systemic disease such as diabetes, multiple sclerosis, kidney disease and atherosclerosis may also result in increased vascular permeability. Compounds of the present invention are also useful for inhibiting vascular permeability triggered by local tissue/organ ischemic (hypoxic) event outside of the central nervous system, including, but not limited to myocardial ischemia and ischemic bowel disease.

Compounds of the present invention provide neuroprotection in a patient. Neuroprotection, as used herein, refers to the protection of neural cells against cell death or apoptosis. One measure of the extent of cell death or apoptosis is infarct volume; the volume of necrotic or dead brain tissue. Imaging techniques and the patient's clinical status can be used to assess infarct volume following an ischemic event. Compounds of the present invention reduce infarct volume of a patient as compared to typical infarct volume experienced in similar ischemic events in the absence of agents of the present invention.

Compounds of the present invention prevent, reduce or inhibit neurodegeneration and/or neurotoxicity associated with vascular permeability that result in symptoms including, but not limited to, visual impairment such as sudden vision loss or diplopia, speech or language impairment such as aphasia or dysarthia, memory impairment, cognitive impairment or dysfunction ranging from mild cognitive impairment to dementia, and motor impairment including, but not limited to, parathesia, loss of muscle control, weakness, numbness or paralysis. Neurological deficits such as those described above, resulting from injury or disease described above may be inhibited or prevented in accordance with the present invention. Thus, the present invention provides the use of compounds of Formula I in the manufacture of a medicament for treating, preventing, inhibiting or alleviating conditions associated with vascular leakage or permeability listed above in a mammal, preferably in a human.

Also encompassed by the present invention are pharmaceutical compositions for treating or modulating vascular permeability comprising at least one compound of Formula I, mixtures thereof, and or pharmaceutical salts thereof, and a pharmaceutically acceptable carrier therefore. Such compositions are prepared in accordance with acceptable pharmaceutical procedures, such as described *in* Remingtons Pharmaceutical Sciences, 17th edition, ed. Alfonoso R. Gennaro, Mack Publishing Company, Easton, PA (1985). Pharmaceutically acceptable carriers are those that are compatible with the other ingredients in the formulation and biologically acceptable.

Liquid carriers may be used in preparing solutions, suspensions, emulsions, syrups and elixirs including intravenous solutions. The active ingredient of this invention can be dissolved or suspended in a pharmaceutically acceptable liquid carrier such as water, organic solvent, or a mixture of both. The liquid carrier can contain other suitable pharmaceutical additives such as solubilizers, emulsifiers, buffers, preservatives, sweeteners, flavoring agents, suspending agents, thickening agents, colors, viscosity regulators, stabilizers, osmo-regulators, antioxidants and antifoaming agents.

Suitable examples of liquid carriers for oral, intravenous and parenteral administration include water (particularly containing additives as above e.g. cellulose derivatives, preferably sodium carboxymethyl cellulose solution), saline, dextrose solutions, dextrose-saline and dextrose-water solutions, alcohols (including monohydric alcohols and polyhydric alcohols e.g. glycols) and their derivatives. Liquid carriers are used in sterile form for parenteral and intravenous administration. PH of liquid formulations may be adjusted in some cases by the addition of HCl, sodium hydroxide, and phosphoric acid. Preferably compositions of the present invention are liquid pharmaceutical compositions which are sterile solutions or suspensions in an iso-osmotic, physiologically compatible buffered system.

Liquid pharmaceutical compositions of the present invention can be administered by, for example, intramuscular, intraperitoneal, intravenous, or subcutaneous injection. Pharmaceutical compositions of the present invention are preferably administered to a patient by intraperitoneal or intravenous injection. Most preferably, the composition is administered intravenously such as by intravenous bolus injection, intravenous i.v. drip, repeated slow bolus administration or infusion.

Oral administration may be either liquid or solid composition form. The compounds of this invention may also be administered orally or parentally, neat or in combination with conventional pharmaceutical carriers. Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid, which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Preferably the pharmaceutical composition is in unit dosage form, e.g. as tablets, capsules, powders, solutions, suspensions, emulsions, granules, suppositories, ampule, or bolus. In such form, the composition is sub-divided in unit dose containing appropriate quantities of the active ingredient; the unit dosage forms can be packaged compositions, for example packeted powders, lyophilyzed powder or cake in ampoules or vials, or vials, ampoules, prefilled syringes or sachets containing liquids. The unit dosage form can be, for example, capsule or tablet itself, or it can be the appropriate number of any such compositions in package form.

The dose provided to a patient will vary depending upon what is being administered, the purpose of the administration, such as prophylaxis or therapy, and the state of the patient, the manner of administration, and the like. A "therapeutically effective amount" is an amount sufficient to cure or ameliorate symptoms of a disease or injury. Generally, a single dose (or dosage form) will contain from about 1 mg/kg to about 30 mg/kg, and more preferably from about 1 mg/kg to about 10 mg/kg of compound of the present invention. It is expected that some patients will receive multiple doses. The dosage to be used in the treatment of a specific case must be subjectively determined by the attending physician. The variables involved include the specific condition and the size, age and response pattern of the patient.

The present invention provides advantages over previously known treatments for stroke and other conditions associated with vascular permeability. In particular, while it is preferable to treat patients as soon as possible after an ischemic injury, compounds of the present invention may be effective in preventing neurodegeneration and development of neurological deficits in some patients when administered 6-18 hours after injury and even up to about 18-24 hours after ischemic injury. Furthermore, treatment may continue and improvement in a patient's prognosis may result from continuous or repeated administration of compound of the present invention for up to about 72 hours or longer following ischemic injury.

Provide as used herein means either directly administering a compound or composition of the present invention, or administering a prodrug, derivative or analog which will form an equivalent amount of the active compound or substance within the body.

This invention will be more fully described in conjunction with the following specific examples which are not to be construed as limiting the scope of this invention.

### Reference Example 1

### 2-Cyano-N-(2,4-dichloro-5-methoxyphenyl)acetamide

2,4-Dichloro-5-methoxyaniline (5.00 g, 26 mmol) and cyanoacetic acid (2.28 g, 26.8 mmol) were mixed in 50 mL of tetrahydrofuran, under N₂, until a solution formed. This solution was heated to reflux and 1,3-diisopropylcarbodiimide (4.2 mL, 26.8 mmol) was added dropwise. After 30 minutes a TLC check (5% MeOH in CH₂Cl₂) indicated that the reaction was complete. The mixture was cooled to ~15°C in an ice-bath. The solid was collected by filtration and washed with tetrahydrofuran. The filtrate was slowly poured into water and stirred for 30 minutes. The white solid was collected by filtration, washed with water and then dissolved in 500 mL of ethyl acetate. The solution was dried over Na₂SO₄ and concentrated in vacuo to give 5.9 g (88%) of 2-cyano-N-(2,4-dichloro-5-methoxyphenyl)acetamide as a white solid, mp 180-181°C; MS 257.0, 259.0 (M-H)-.

| Analysis for C₁₀H₈Cl₂N₂O₂: | | | |
|---|---|---|---|
| Calcd: | C, 46.36; | H, 3.11; | N, 10.81. |
| Found: | C, 46.25; | H, 3.10; | N, 10.85. |

### Reference Example 2

### 2-Cyano-N-(2,4-dichloro-5-methoxyphenyl)-3-[(3-iodo-4-methoxylphenyl)amino]-prop-2-enamide

To a suspension of 2-cyano-N-(2,4-dichloro-5-methoxyphenyl)acetamide (5.00 g, 19.30 mol) in 400 mL of *iso*-propanol, under N₂, is added 3-iodo-*p*-anisidine (5.80 g, 23.16 mmol). This mixture is heated to reflux to give a clear yellow solution. To this solution, triethylorthoformate (8.60 mL, 52.11 mmol) is added dropwise and the reaction mixture is heated at reflux overnight. An additional 10 mL of triethylorthoformate is added and the mixture is heated at reflux overnight. The mixture is allowed to cool to room temperature and the white solid is collected by filtration, washing with *iso*-propanol, and dried overnight at ~40°C under reduced pressure. Purification by suspension in hot ethyl acetate followed by addition of cold hexanes gives 8.50 g (85%) of 2-cyano-N-(2,4-dichloro-5-methoxyphenyl)-3-[(3-iodo-4-methoxyphenyl)amino]prop-2-enamide as a yellow solid, mp 289-290 °C; MS (ES) m/z 516.7 (M-H)-.

| Analysis for C₁₈H₁₄Cl₂lN₃O₃: | | | |
|---|---|---|---|
| Calcd: | C, 41.73; | H, 2.72; | N, 8.11. |
| Found: | C, 40.88; | H, 2.64; | N, 7.90. |

### Reference Example 3

### 4-[(2,4-Dichloro-5-methoxyphenyl)amino]-7-iodo-6-methoxy-3-quinolinecarbonitrile

To a suspension of 2-cyano-N-(2,4-dichloro-5-methoxyphenyl)-3-[(3-iodo-4-methoxyphenyl)amino]prop-2-enamide (720 mg, 1.39 mmol) in 40 mL of acetonitrile is added 0.2 mL of methanol. The mixture is heated to reflux and phosphorous oxychloride (1.24 mL, 13.9 mmol) is added dropwise, via syringe. This solution is heated at reflux overnight. After 24 hours, the mixture is cooled in an ice-bath and the solid is collected by filtration, washing with cold acetonitrile (40 mL) and then suspended in tetrahydrofuran (100 mL). To both the acetonitrile filtrate and the tetrahydrofuran suspension are added concentrated ammonium hydroxide (2x50 mL) and the mixtures are stirred for 1 hour. Water (2x 800 ml) is added and stirring is continued for 2 hours. The resulting solids are combined, washed with hot water and dried under reduced pressure at ∼ 40°C, overnight, to provide 200 mg (29%) of 4-[(2,4-dichloro-5-methoxyphenyl)amino]-7-iodo-6-methoxy-3-quinolinecarbonitrile, as yellow solid, mp 253-254 °C; ¹H NMR (400 MHz, DMSO-*d*₆) δ 3.86 (s, 3H), 4.00 (s, 3H), 7.33(s, 1 H), 7.74(s, 1H). 7.86 (s, 1 H), 8.39 (s, 1 H), 8.43 (s, 1 H), 9.61 (s, 1 H); MS (ES) *m*/*z* 500.0, 502.1 (M+H)+.

| Analysis for C₁₈H₁₂Cl₂IN₃O₂- H₂O: | | | |
|---|---|---|---|
| Theory: | C, 41.72, | H, 2.72, | N, 8.11 |
| Found: | C, 41.80; | H, 2.52; | N, 7.87. |

### Reference Example 4

### 1-Ethoxy-2-iodo-4-nitrobenzene

A suspension of 2-iodo-4-nitrophenol (21 g, 79.2 mmol) [Kometani, T.; et. al., Tetrahedron Lett. (1985), 26(17), 2043], ethyl iodide (9 mL, 0.48 mol) and potassium carbonate (40.7 g, 0.3 mol) in 100 mL of N, N-dimethylformamide is heated at 70°C for 3 h. The reaction is cooled to room temperature and ethyl acetate is added. The inorganic salts are filtered and washed with ethyl acetate. The organic material is washed with water (3X) and brine, dried over magnesium sulfate and filtered. Upon concentration of the filtrate a solid appears. This solid is filtered and washed with hexanes to give 5.2 g of 1-ethoxy-2-iodo-4-nitrobenzene as white crystals. Concentration of the filtrate provides an additional 11.3 g of the desired product, mp 81-83°C; ¹H NMR (400 MHz, DMSO-*d*₆) δ1.42 (t, 3H), 4.26 (q, 2H), 7.18 (d, 1H), 8.26 (dd, 1H), 8.55 (d, 1H).

| Analysis for C₈H₈INO₃: | | | |
|---|---|---|---|
| Theory: | C, 32.79, | H, 2.75, | N, 4.78. |
| Found: | C, 32.71, | H, 2.58, | N, 4.53. |

### Reference Example 5

### (4-Ethoxy-3-iodophenyl)amine

A suspension of iron (3.81 g, 70 mmol) and ammonium chloride (5.47 g, 102 mmol) in 80 mL of ethanol and 25 mL of water is heated to reflux. 1-Ethoxy-2-iodo-4-nitrobenzene (5.0 g, 20 mmol) is added in portions and the reaction is heated at reflux for 1 h. The hot mixture is filtered through Celite, washing with hot ethanol. The filtrate is concentrated in vacuo and treated with ethyl acetate and water. The organic layer is extracted, washed with brine, dried over magnesium sulfate and filtered. Removal of the solvent in vacuo provides 5.1 g of (4-ethoxy-3-iodophenyl)amine as a light brown oil; ¹H NMR (400 MHz, DMSO-*d*₆) δ1.30 (s, 3H), 3.89 (q, 2H), 4.82 (bs, 2H), 6.53 (dd, 1 H), 6.72 (d, 1 H), 7.11 (d, 1 H); MS (ES) *m*/*z* 263.9 (M+H)+.

| Analysis for C₈H₁₀INO: | | | |
|---|---|---|---|
| Theory: | C, 36.52, | H, 3.83, | N, 5.32. |
| Found: | C, 36.84, | H, 3.71, | N, 4.96. |

### Reference Example 6

### 2-Cyano-N-(2,4-dichloro-5-methoxyphenyl)-3-[(4-ethoxy-3-iodophenyl)amino]acrylamide

To a suspension of 2-cyano-N-(2,4-dichloro-5-methoxyphenyl)acetamide (5.44 g, 21.0 mmol) in 350 mL of *iso*-propanol, under N₂, is added (4-ethoxy-3-iodophenyl)-amine (5.0 g, 19.30 mmol). This mixture is heated to reflux and triethylorthoformate (8.53 mL, 51.30 mmol) is added dropwise and the reaction mixture is heated at reflux overnight. The mixture is allowed to cool to room temperature and the yellow solid is collected by filtration, washing with *iso*-propanol, and dried overnight at ~40°C under reduced pressure to give 5.46 g (54%) of 2-cyano-N-(2,4-dichloro-5-methoxyphenyl)-3-[(4-ethoxy-3-iodophenyl)amino]prop-2-enamide as a yellow solid, mp >245 °C; MS (EI) *m*/*z* 531.01 (M)+.

| Analysis for C₁₉H₁₆Cl₂IN₃O₃: | | | |
|---|---|---|---|
| Calcd: | C, 42.88; | H, 3.03; | N, 7.90. |
| Found: | C, 42.99; | H, 2.97; | N, 7.74. |

### Reference Example 7

### 4-[(2,4-Dichloro-5-methoxyphenyl)amino]-6-ethoxy-7-iodo-3-quinolinecarbonitrile

A suspension of 2-cyano-N-(2,4-dichloro-5-methoxyphenyl)-3-[(4-ethoxy-3-iodophenyl)amino]prop-2-enamide (2.0 g, 3.76 mmol) in 100 mL of toluene is heated to reflux and phosphorous oxychloride (3.5 mL, 37.6 mmol) is added dropwise via syringe. This suspension is heated at reflux for 6 hours and additional phosphorous oxychloride (3.5 mL, 37.6 mmol) is added to slowly give a dark solution. After 72 hours the mixture is cooled to room temperature, the solid is filtered and washed with toluene and ether. The light brown solid is dried under reduced pressure at - 40°C, overnight, to provide 1.47 g (76%) of 4-[(2,4-dichloro-5-methoxyphenyl)amino]-6-ethoxy-7-iodo-3-quinolinecarbonitrile as a yellow solid, mp 213-215 °C; ¹H NMR (300 MHz, DMSO-*d*₆) δ 1.48 (t, 3H, J = 6.9 Hz), 4.32 (q, 2H, J = 6.9 Hz), 3.88 (s, 3H), 7.53 (s, 1 H), 7.87 (s, 1H), 8.06 (s, 1H), 8.49 (s, 1H), 9.02 (s, 1H), 11.14 (bs, 1H); MS (ES) m/z 514.1 (M+H)+.

| Analysis for C₁₉H₁₄Cl₂IN₃O₂- 4.0 HCl: | | | |
|---|---|---|---|
| Theory: | C, 34.58, | H, 2.75, | N, 6.37 |
| Found: | C, 34.79; | H, 2.60; | N, 6.13. |

### Reference Example 8

### 4-[(2,4-Dichloro-5-methoxyphenyl)amino]-7-(4-hydroxybut-1-ynyl)-6-methoxy-3-quinolinecarbonitrile

A mixture of 3-cyano-4-[(2,4-dichloro-5-methoxyphenyl)anilino]-6-methoxy-7-quinolinyl trifluoromethanesulfonate (2.0 g, 3.8 mmol), 3-butyn-1-ol (0.44 mL, 0.56 mmol), tetrakis(triphenylphosphine)palladium (0.22 g, 0.19 mmol) and copper iodide (45 mg, 0.24 mmol) in a mixture of 12 mL of triethylamine and 30 mL of 1,4-dioxane is heated at 95-100°C for 2 hours and then cooled to room temperature. Ethyl acetate and water are added. The solids are removed by filtration, triturated in hot diethyl ether, filtered and dried to provide 0.65 g (38%) of 4-[(2,4-dichloro-5-methoxyphenyl)amino]-7-(4-hydroxybut-1 -ynyl)-6-methoxy-3-quinolinecarbonitrile, mp 230-232 °C; ¹H NMR (DMSO-*d*₆) δ 2.65 (t, 2H, J = 6.9 Hz), 3.62 (q, 2H, J = 6.9, 6.0 Hz), 3.86 (s, 3H), 3.98 (s, 3H), 4.96 (t, 1H, J = 6.0 Hz), 7.39 (s, 1H), 7.76 (s, 1 H), 7.89 (s, 2H), 8.44 (s, 1H), 9.97 (bs, 1 H); MS (ES) *m*/*z* 442.0 (M+H)+; HRMS 442.07198 (M+H)+; HPLC - 93%.

### Reference Example 9

### 4-[(2,4-Dichloro-5-methoxyphenyl)amino]-6-ethoxy-7-(4-hydroxybut-1-ynyl)-3-quinolinecarbonitrile

A mixture of 4-[(2,4-dichloro-5-methoxyphenyl)amino]-6-ethoxy-7-iodo-3-quinoline-carbonitrile (0.6 g, 1.2 mmol), 3-butyn-1-ol (0.13 mL, 1.7 mmol), tetrakis(triphenylphosphine)palladium (68 mg, 0.058 mmol) and copper iodide (15 mg, 0.076 mmol) in a mixture of 4 mL of triethylamine and 10 mL of 1,4-dioxane is heated at 95 °C for 3 hours and then cooled to room temperature. Ethyl acetate and water are added. The organic layer is extracted, dried over magnesium sulfate, filtered and concentrated. Ether is added and the solid is filtered and washed with hot diethyl ether (3X). The solid and filtrate are combined and purified by chromatography using a gradient of 0 to 10 % of methanol and dichloromethane. The yellow solid obtained is triturated in hot diethyl ether, filtered and dried to provide 0.16 g (31%) of 4-[(2,4-dichloro-5-methoxyphenyl)amino]-6-ethoxy-7-(4-hydroxybut-1-ynyl)-3-quinoline-carbonitrile, mp 220-222 °C; ¹H NMR (DMSO-*d*₆) δ 1.44 (t, 3H, J = 6.8 Hz), 2.65 (t, 2H, J = 6.8 Hz), 3.63 (dt, 2H, J = 6.9, 5.8 Hz), 3.86 (s, 3H), 4.25 (q, 2H, J = 6.8 Hz), 4.95 (t, 1H, J = 5.8 Hz), 7.39 (s, 1 H), 7.77 (s, 1H). 7.87 (s, 2H), 8.44 (s, 1 H), 9.75 (bs, 1 H); MS (ES) *m*/*z* 456.1 (M+H)+; HRMS 456.08763 (M+H)+; HPLC - 98%.

### Example 1

### 4-[(2,4-Dichloro-5-methoxyphenyl)amino]-7-[4-(dimethylamino)but-1-ynyl]-6-methoxy-3-quinolinecarbonitrile

To a mixture of 4-[(2,4-dichloro-5-methoxyphenyl)amino]-7-(4-hydroxybut-1-ynyl)-6-methoxy-3-quinolinecarbonitrile (0.3 g, 0.68 mmol) and triethylamine (0.47 mL, 3.4 mmol) in 3 mL of N,N-dimethylformamide and 15 mL of tetrahydrofuran at 0 °C was added dropwise methanesulfonyl chloride (0.16 mL, 2.0 mmol). The mixture is stirred for 1.25 hours and dimethylamine (2.0 mL, 2.0 M solution in tetrahydrofuran) is added. The reaction is stirred at room temperature overnight. An additional 3.0 mL of triethylamine are added and the reaction is stirred at room temperature overnight. The mixture is concentrated and diluted with ethyl acetate and water. The organic layer is extracted, dried over magnesium sulfate, filtered and concentrated. The residue is purified by chromatography using a gradient of 0 to 10% of methanol in dichloromethane. The product is triturated in hot diethyl ether, filtered and dried to provide 62 mg (20%) of 4-[(2,4-dichloro-5-methoxyphenyl)amino]-7-[4-(dimethylamino)but-1-ynyl]-6-methoxy-3-quinolinecarbonitrile as a yellow solid, mp 177-179 °C; ¹H NMR (DMSO-*d*₆) δ 2.22 (s, 6H), 2.56 (t, 2H, J = 6.8 Hz), 2.65 (t, 2H, J = 6.8 Hz), 3.86 (s, 3H), 3.96 (s, 3H), 7.38 (s, 1H), 7.76 (s, 1 H), 7.88 (s, 2H), 8.43 (s, 1 H), 9.82 (bs, 1 H); MS (ES) m/z 469.1 (M+H)+.

| Analysis for C₂₄H₂₂Cl₂N₄O₂-1.5 H₂O | | | |
|---|---|---|---|
| Theory: | C, 58.07, | H, 5.08, | N, 11.29. |
| Found: | C, 58.46, | H, 4.82, | N, 10.99. |

### Example 2

### 4-[(2,4-Dichloro-5-methoxyphenyl)amino]-6-methoxy-7-[4-(4-methylpiperazin-1-yl)but-1-ynyl]-3-quinolinecarbonitrile

The same procedure used for the synthesis of Example 1 is followed to provide 90 mg (25%) of 4-[(2,4-dichloro-5-methoxyphenyl)amino]-6-methoxy-7-[4-(4-methyl-piperazin-1-yl)but-1-ynyl]-3-quinolinecarbonitrile as a yellow solid, mp 165-167 °C; ¹H NMR (DMSO-*d*₆) δ 2.17 (s, 3H), 2.35 (bs, 4H), 2.50 (bs, 4H), 2.60-2.68 (m, 4H), 3.86 (s, 3H), 3.96 (s, 3H), 7.34 (s, 1 H), 7.74 (s, 1H), 7.83 (s, 1 H), 7.88 (s, 1H), 8.43 (s, 1 H), 9.83 (bs, 1 H), MS (ES) m/z 524.2 (M+H)+.

| Analysis for C₂₇H₂₇Cl₂N₅O₂ - 1.5 H2O | | | |
|---|---|---|---|
| Theory: | C, 58.80, | H, 5.48, | N, 12.70. |
| Found: | C, 58.89, | H, 5.19, | N, 12.38. |

### Alternative Preparation of Example 2:

A mixture of 3-cyano-4-[(2,4-dichloro-5-methoxyphenyl)anilino]-6-methoxy-7-quinolinyl trifluoromethanesulfonate (2.5 g, 4.8 mmol), 1-but-3-ynyl-4-methylpiperazine (1.8 g, 11.8 mmol) [Vaillancourt, V. A. et. al. WO2002002558A1] bis(triphenylphosphine)palladium dichloride (170 mg, 0.22 mmol), potassium carbonate (3.31 g, 0.024 mol), triphenylphosphine (25 mg, 0.9 mmol) and copper iodide (45 mg, 0.22 mmol) in a mixture of 6 mL of methanol and 30 mL of tetrahydrofuran is heated at 60 °C for 4 hours and then cooled to room temperature. Ethyl acetate and water are added. The organic layer is extracted, dried over magnesium sulfate, filtered and concentrated. Purification of the residue by chromatography using a gradient of 0 to 10% of methanol in dichloromethane, followed by 20% of methanol-dichloromethane/1% NH₄OH as the solvent system provides 2.2 g (88%) of 4-[(2,4-dichloro-5-methoxyphenyl)amino]-6-methoxy-7-[4-{4-methylpiperazin-1-yl)but-1-ynyl]-3-quinolinecarbonitrile.

### Second Alternative Preparation of Example 2

A mixture of 4-[(2,4-dichloro-5-methoxyphenyl)amino]-7-iodo-6-methoxy-3-quinoline-carbonitrile (0.2 g, 0.4 mmol), 1-but-3-ynyl-4-methylpiperazine (0.21 g, 1.4 mmol) [Vaillancourt, V. A. et. al. WO2002002558A1], bis(triphenylphosphine)palladium dichloride (14 mg, 0.02 mmol), triphenylphosphine (21 mg, 0.08 mmol) and copper iodide (5 mg, 0.02 mmol) in a mixture of 2 mL of triethylamine and 1 mL of N-methylpyrrolidinone is heated at 70°C for 4 hours and then cooled to room temperature. Ethyl acetate and water are added. The organic layer is extracted, dried over magnesium sulfate, filtered and concentrated. Purification of the residue by chromatography using a gradient of 0 to 10% methanol in dichloromethane, followed by 15% of methanol-dichloromethane/1% NH₄OH as the solvent system provides 78 mg (37%) of 4-[(2,4-dichloro-5-methoxyphenyl)amino]-6-methoxy-7-[4-(4-methyl-piperazin-1-yl)but-1-ynyl]-3-quinolinecarbonitrile.

### Example 3

### 4-[(2,4-Dichloro-5-methoxyphenyl)amino]- 6-methoxy-7-(4-morpholin-4-ylbut-1-ynyl)-3-quinolinecarbonitrile

The same procedure used for the synthesis of Example 1 is followed to provide 150 mg (43%) of 4-[(2,4-dichloro-5-methoxyphenyl)amino]-6-methoxy-7-(4-morpholin-4-ylbut-1-ynyl)-3-quinolinecarbonitrile as a yellow solid, mp 140-142 °C; ¹H NMR (DMSO-*d*₆) δ 2.50 (t, 4H, J = 4.4 Hz), 2.58-2.73 (m, 4H), 3.60 (t, 4H, J = 4.4 Hz), 3.87 (s, 3H), 3.98 (s, 3H), 7.39 (s, 1 H), 7.77 (s, 1H), 7.87 (s, 1H). 7.89 (s, 1H). 8.45 (s, 1 H), 9.82 (bs, 1 H); MS (ES) *m*/*z* 511.0 (M+H)+.

| Analysis for C₂₆H₂₄Cl₂N₄O₃ | | | |
|---|---|---|---|
| Theory: | C, 61.06, | H, 4.73, | N, 10.96. |
| Found: | C, 60.89, | H, 4.74, | N, 10.82. |

### Example 4

### 4-[(2,4-Dichloro-5-methoxyphenyl)amino]- 6-methoxy-7-(4-piperazin-1-ylbut-1-ynyl)-3-quinolinecarbonitrile

The same procedure used for the synthesis of Example 1 is followed. After purification by column chromatography 120 mg of a mixture containing the product is obtained. The mixture is further purified by HPLC using a gradient of 5 to 95% acetonitrile/water (0.02%TFA) to provide 15 mg (5%) of 4-[(2,4-dichloro-5-methoxyphenyl)amino]-6-methoxy-7-(4-piperazin-1-ylbut-1-ynyl)-3-quinolinecarbonitrile as a yellow solid, mp 188-190 °C; ¹H NMR (DMSQ-*d*₆) δ 2.85 (t, 2H, J = 6.8 Hz), 3.03 (bs, 6H), 3.32 (bs, 4H), 3.89 (s, 3H), 3.98 (s, 3H), 7.35 (s, 1H), 7.77 (s, 1H), 7.89 (s, 1H), 7.92 (s, 1 H), 8.52 (s, 1 H), 8.81 (bs, 1H), 10.01 (bs, 1 H); MS (ES) *m*/*z* 510.1 (M+H)+.

| Analysis for C₂₆H₂₅Cl₂N₅O₂ - 3 TFA/1 H₂O | | | |
|---|---|---|---|
| Theory: | C, 44.18, | H, 3.48, | N, 8.06. |
| Found: | C, 44.07, | H, 3.22, | N, 8.06. |

### Example 5

### 4-[(2,4-Dichloro-5-methoxyphenyl)amino]-6-ethoxy-7-[4-(4-methylpiperazin-1-yl)but-1-ynyl]-3-quinolinecarbonitrile

The same procedure used for the synthesis of Example 1 is followed to provide 45 mg (24%) of 4-[(2,4-dichloro-5-methoxyphenyl)amino]-6-ethoxy-7-[4-(4-methyl-piperazin-1-yl)but-1-ynyl]-3-quinolinecarbonitrile as a yellow solid, mp 158-160°C; ¹H NMR (DMSO-*d*₆) δ 1.43 (t, 3H, J = 6.8 Hz), 2.19 (s, 3H), 2.38 (bs, 4H), 2.50 (bs, 4H), 2.60-2.67 (m, 4H), 3.86 (s, 3H), 4.22 (q, 2H, J = 6.8 Hz), 7.36 (s, 1H), 7.74 (s, 1H), 7.84 (s, 1H), 7.88 (s, 1 H), 8.42 (s, 1H), 9.78 (bs, 1 H); MS (ES) m/z 538.2 (M+H)+; HRMS 538.17726 (M+H)+; HPLC purity is 99%.

## Claims

1. A compound having the structure: 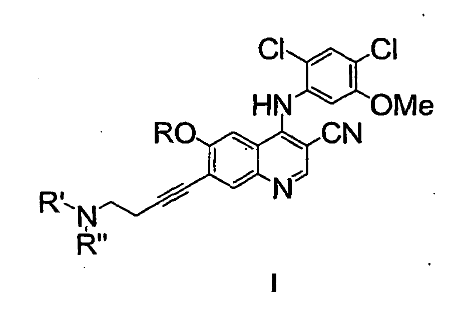 wherein:
R is methyl or ethyl;
R' and R" are independently alkyl of 1 to 3 carbon atoms, or R' and R", taken together with the nitrogen to which they are attached, can form a 5 or 6 membered saturated ring which may optionally contain an additional heteroatom selected from NR"', O or S(O)ₙ;
n is 0-2;
R'" is hydrogen or alkyl of 1 to 3 carbon atoms; and pharmaceutically acceptable salts thereof.

2. The compound of Claim 1 wherein R' and R" are each methyl.

3. The compound of Claim 1 wherein R' and R", taken together with the nitrogen to which they are attached, form a N-(C₁-C₃)-alkylpiperazine, piperazine or morpholine ring.

4. The compound of Claim 3 wherein the N-(C₁-C₃)alkylpiperazine ring is N-methyl-piperazine.

5. The compound of any one of Claims 1 to 4 wherein R is methyl.

6. The compound of Claim 1 wherein the compound is selected from:
4-[(2,4-Dichloro-5-methoxyphenyl)amino]-7-[4-(dimethylamino)but-1-ynyl]-6 methoxy-3-quinolinecarbonitrile;
4-[(2,4-Dichloro-5-methoxyphenyl)amino]- 6-methoxy-7-[4-(4-methylpiperazin-1-yl)but-1-ynyl]-3-quinolinecarbonitrile;
4-[(2,4-Dichloro-5-methoxyphenyl)amino]- 6-methoxy-7-(4-morpholin-4-ylbut-1-ynyl)-3-quinolinecarbonitrile;
4-[(2,4-Dichloro-5-methoxyphenyl)amino]- 6-methoxy-7-(4-piperazin-1-ylbut-1-ynyl)-3-quinolinecarbonitrile; and
4-[(2,4-Dichloro-5-methoxyphenyl)amino]- 6-ethoxy-7-[4-(4-methylpiperazin-1-yl)but-1-ynyl]-3-quinolinecarbonitrile;
and pharmaceutically acceptable salts thereof.

7. A pharmaceutical composition comprising a compound according to any one of claims 1 to 6 and pharmaceutically acceptable salts thereof, and at least one pharmaceutically acceptable carrier or excipient.

8. A pharmaceutical composition comprising a vascular permeability inhibiting amount of a compound according to any one of claims 1 to 6 and pharmaceutically acceptable salts thereof, and at least one pharmaceutically acceptable carrier or excipient.

9. The composition of Claim 7 or 8 in an intravenous dosage form.

10. The use of a compound according to any one of claims 1 to 6 in the manufacture of a medicament for providing neuroprotection in a patient following a cerebrovascular ischemic event, inhibiting neurological deficits in a patient following a cerebrovascular ischemic event, reducing infarct volumes in a patient following a cerebrovascular ischemic event, or inhibiting post-ischemic vascular permeability of cerebral blood vessels in a patient suffering from a cerebrovascular event.

11. The use of claim 10 wherein the patient is a human.

12. The use of claim 10 or claim 11 wherein the ischemic event is transient.

13. The use of claim 10 or claim 11 wherein the ischemic event is acute.

14. The use of claim 13 wherein the ischemic event is stroke, head trauma, spinal trauma, general anoxia, or hypoxia.

15. The use of any one of claims 10 to 14 wherein the ischemic event occurs during cerebral hemmorhage, perinatal asphyxia, cardiac arrest or status epilepticus.

## Patentansprüche

1. Verbindung mit der Struktur: 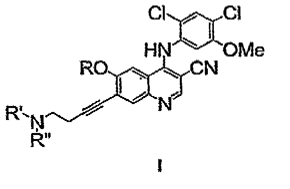 wobei:
R Methyl oder Ethyl ist;
R' und R" unabhängig voneinander Alkyl mit 1 bis 3 Kohlenstoffatomen sind, oder R' und R" zusammen genommen mit dem Stickstoffatom, an das sie gebunden sind, einen 5- oder 6-gliedrigen gesättigten Ring bilden können, der optional ein zusätzliches Heteroatom enthalten kann, das aus NR"', O oder S(O)ₙ ausgewählt wird;
n 0-2 ist;
R'" Wasserstoff oder Alkyl mit 1 bis 3 Kohlenstoffatomen ist;
und pharmazeutisch annehmbare Salze davon.

2. Verbindung nach Anspruch 1, wobei R' und R" jeweils Methyl sind.

3. Verbindung nach Anspruch 1, wobei R' und R" zusammen genommen mit dem Stickstoffatom, an das sie gebunden sind, einen N-(C₁-C₃)-Alkylpiperazin-, Piperazin- oder Morpholin-Ring bilden.

4. Verbindung nach Anspruch 3, wobei der N-(C₁-C₃)-Alkylpiperazin-Ring N-Methyl-piperazin ist.

5. Verbindung nach irgendeinem der Ansprüche 1 bis 4, wobei R Methyl ist.

6. Verbindung nach Anspruch 1, wobei die Verbindung ausgewählt wird aus:
4-[(2,4-Dichlor-5-methoxyphenyl)amino]-7-[4-(dimethylamino)but-1-inyl]-6-methoxy-3-chinolincarbonitril;
4-[(2,4-Dichlor-5-methoxyphenyl)amino]-6-methoxy-7-[4-(4-methylpiperazin-1-yl)but-1-inyl]-3-chinolincarbonitril;
4-[(2,4-Dichlor-5-methoxyphenyl)amino]-6-methoxy-7-(4-morpholin-4-ylbut-1-inyl)-3-chinolincarbonitril;
4-[(2,4-Dichlor-5-methoxyphenyl)amino]-6-methoxy-7-(4-piperazin-1-ylbut-1-inyl)-3-chinolincarbonitril; und
4-[(2,4-Dichlor-5-methoxyphenyl)amino]-6-ethoxy-7-[4-(4-methylpiperazin-1-yl)but-1-inyl]-3-chinolincarbonitril;
und pharmazeutisch annehmbaren Salzen davon.

7. Pharmazeutische Zusammensetzung umfassend eine Verbindung nach irgendeinem der Ansprüche 1 bis 6 und pharmazeutisch annehmbare Salze davon sowie mindestens einen pharmazeutisch annehmbaren Träger oder Hilfsstoff.

8. Pharmazeutische Zusammensetzung umfassend eine Gefäßpermeabilität hemmende Menge einer Verbindung nach irgendeinem der Ansprüche 1 bis 6 und pharmazeutisch annehmbarer Salze davon sowie mindestens einen pharmazeutisch annehmbaren Träger oder Hilfsstoff,

9. Zusammensetzung nach Anspruch 7 oder 8 in einer intravenösen Dosierungsform.

10. Verwendung einer Verbindung nach irgendeinem der Ansprüche 1 bis 6 bei der Herstellung eines Arzneimittels zur Bereitstellung einer Neuroprotektion bei einem Patienten nach einem zerebrovaskulären ischämischen Ereignis, einer Hemmung neurologischer Defizite bei einem Patienten nach einem zerebrovaskulären ischämischen Ereignis, einer Reduzierung von Infarktvolumina bei einem Patienten nach einem zerebrovaskulären ischämischen Ereignis oder einer Hemmung postischämischer Gefäßpermeabilität von Himblutgefäßen bei einem Patienten, der an einem zerebrovaskulären Ereignis erkrankt ist.

11. Verwendung nach Anspruch 10, wobei der Patient ein Mensch ist.

12. Verwendung nach Anspruch 10 oder Anspruch 11, wobei das ischämische Ereignis vorübergehend ist.

13. Verwendung nach Anspruch 10 oder Anspruch 11, wobei das ischämische Ereignis akut ist.

14. Verwendung nach Anspruch 13, wobei das ischämische Ereignis Schlaganfall, Schädeltrauma, Wirbelsäulenverletzung, allgemeine Anoxie oder Hypoxie ist.

15. Verwendung nach irgendeinem der Ansprüche 10 bis 14, wobei das ischämische Ereignis während einer Hirnblutung, einer perinatalen Asphyxie, eines Herzstillstands oder eines epileptischen Anfalls eintritt.

## Revendications

1. Composé ayant la structure : 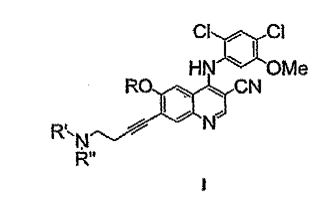 dans laquelle :
R représente un groupe méthyle ou un groupe éthyle ;
R' et R" représentent indépendamment un groupe alkyle de 1 à 3 atomes de carbone, ou R' et R", pris conjointement avec l'atome d'azote auquel ils sont attachés, peuvent former un cycle saturé de 5 ou 6 membres qui peut éventuellement contenir un hétéroatome supplémentaire choisi parmi NR"', O ou S(O)ₙ ;
n vaut 0 à 2 ;
R"' représente un atome d'hydrogène ou un groupe alkyle de 1 à 3 atomes de carbone ; et les sels pharmaceutiquement acceptables de celui-ci.

2. Composé selon la revendication 1, dans lequel R' et R" représentent chacun un groupe méthyle.

3. Composé selon la revendication 1, dans lequel R' et R", pris conjointement avec l'atome d'azote auquel ils sont attachés, forment un cycle N-[alkyle en (C₁-C₃)]-pipérazine, un cycle pipérazine ou un cycle morpholine.

4. Composé selon la revendication 3, dans lequel le cycle N-[alkyle en (C₁-C₃)-pipérazine est la N-méthyl-pipérazine.

5. Composé selon l'une quelconque des revendications 1 à 4, dans lequel R est un groupe méthyle.

6. Composé selon la revendication 1, ledit composé étant choisi parmi :
le 4-[(2,4-dichloro-5-méthoxyphényl)amino]-7-[4-(diméthylamino)but-1-ynyl]-6-méthoxy-3-quinolinecarbonitrile ;
le 4-[(2,4-dichlora-5-méthoxyphényl)amino]-6-méthoxy-7-[4-(4-méthylpipérazin-1-yl)but-1-ynyl]-3-quinolinecarbonitrile;
le 4-[(2,4-dichloro-5-méthoxyphényl)amino]-6-méthoxy-7-(4-morpholin-4-ylbut-1-ynyl)-3-quinolinecarbonitrile;
le 4-[(2,4-dichloro-5-méthoxyphényl)amino]-6-méthoxy-7-(4-pipérazin-1-ylbut-1-ynyl)-3-quinolinecarbonitrile ; et
le 4-[(2,4-dichloro-5-méthoxyphényl)amino]-6-éthoxy-7-[4-(4-méthylpipérazin-1-yl)but-1-ynyl]-3-quinolinecarbonitrile ;
et les sels pharmaceutiquement acceptables de celui-ci.

7. Composition pharmaceutique comprenant un composé selon l'une quelconque des revendications 1 à 6 et les sels pharmaceutiquement acceptables de celui-ci, et au moins un support ou un excipient pharmaceutiquement acceptable.

8. Composition pharmaceutique comprenant une quantité inhibant la perméabilité vasculaire d'un composé selon l'une quelconque des revendications 1 à 6 et les sels pharmaceutiquement acceptables de celui-ci, et au moins un support ou un excipient pharmaceutiquement acceptable.

9. Composition selon la revendication 7 ou 8 sous une forme posologique intraveineuse.

10. Utilisation d'un composé selon l'une quelconque des revendications 1 à 6 dans la fabrication d'un médicament pour procurer une neuroprotection chez un patient après un événement vasculaire ischémique cérébral, pour inhiber des déficits neurologiques chez un patient après un événement vasculaire ischémique cérébral, pour réduire des volumes d'infarctus chez un patient après un événement vasculaire ischémique cérébral, ou pour inhiber la perméabilité vasculaire post-ischémique de vaisseaux sanguins cérébraux chez un patient souffrant d'un événement vasculaire cérébral.

11. Utilisation selon la revendication 10 dans laquelle le patient est un humain.

12. Utilisation selon la revendication 10 ou la revendication 11, dans laquelle l'événement ischémique est transitoire.

13. Utilisation selon la revendication 10 ou la revendication 11, dans laquelle l'événement ischémique est aigu.

14. Utilisation selon la revendication 13, dans laquelle l'événement ischémique est une attaque, un traumatisme crânien, un traumatisme médullaire, une anoxie générale, ou une hypoxie.

15. Utilisation selon l'une quelconque des revendications 10 à 14, dans laquelle l'événement ischémique se produit pendant une hémorragie cérébrale, une asphyxie périnatale, un arrêt cardiaque ou un état de mal épileptique.
